# EUROPEAN PATENT SPECIFICATION

(11) **EP 3 380 631 B1**
(45) Date of publication and mention of the grant of the patent: **18.11.2020**
(21) Application number: 16804883.3
(22) Date of filing: 25.11.2016
(51) Int. Cl.: C12Q 1/04, C12Q 1/34

(54) **CARBAPENEMASE DETECTION METHOD**
VERFAHREN ZUR ERKENNUNG VON CARBAPENEMASE
PROCÉDÉ DE DÉTECTION DE CARBAPÉNÉMASE

(30) Priority: 27.11.2015 GB 201520977
(43) Date of publication of application: 03.10.2018
(73) Proprietor: Mast Group Limited, Merseyside L20 1EA (GB)
(72) Inventor: WILSON, Michelle, Wirral Merseyside CH62 9BU (GB)
(74) Representative: Forrest, Stuart
(86) International application number: PCT/GB2016/053716
(87) International publication number: WO 2017/089823

(56) References cited:
- WO-A2-2015/013177
- US-A1- 2014 134 656
- PATRICE NORDMANN ET AL: "Rapid Detection of Carbapenemase-producing Enterobacteriaceae", EMERGING INFECTIOUS DISEASES, vol. 18, no. 9, 9 September 2012 (2012-09-09), pages 1503-1507, XP055251024, US ISSN: 1080-6040, DOI: 10.3201/eid1809.120355
- SAMEH ABDELGHANI ET AL: "Comparison of the Carba NP, Modified Carba NP, and Updated Rosco Neo-Rapid Carb Kit Tests for Carbapenemase Detection", JOURNAL OF CLINICAL MICROBIOLOGY, vol. 53, no. 11, 26 August 2015 (2015-08-26), pages 3539-3542, XP055336839, US ISSN: 0095-1137, DOI: 10.1128/JCM.01631-15
- LAURENT DORTET ET AL: "Evaluation of the RAPIDEC CARBA NP, the Rapid CARB Screen and the Carba NP test for biochemical detection of carbapenemase-producing Enterobacteriaceae", JOURNAL OF ANTIMICROBIAL CHEMOTHERAPY., vol. 70, no. 11, 9 August 2015 (2015-08-09), pages 3014-3022, XP055336858, GB ISSN: 0305-7453, DOI: 10.1093/jac/dkv213
- A. Williams, G. Vanstone & I. Balakrishnan: "Direct detection of carbapenemase-producing Enterobacteriaceae and Pseudomonas spp. from positive blood cultures", British Society Antimicrobial Chemotherapy poster , 31 December 2014 (2014-12-31), XP002766070, Retrieved from the Internet: URL:http://www.check-points.eu/downloads/W illiams_et_al.BSAC_poster_2014.pdf [retrieved on 2017-01-18]
- Jeremy M. T. Hamilton-Miller: "Chemical and Microbiologic Aspects of Penems, a Distinct Class of [beta]-Lactams: Focus on Faropenem", Pharmacotherapy : the journal of human pharmacology and drug therapy, vol. 23, no. 11, 1 November 2003 (2003-11-01), pages 1497-1507, XP055571418, US ISSN: 0277-0008, DOI: 10.1592/phco.23.14.1497.31937
- WISE ET AL: "The carbapenems and penem antibiotics-a brief review", ANTIMICROBIC NEWSLETTER,, vol. 7, no. 10, 1 October 1990 (1990-10-01), pages 73-78, XP023935506, ISSN: 0738-1751, DOI: 10.1016/0738-1751(90)90045-E [retrieved on 1990-10-01]

## Description

The present invention relates to a method for detecting the presence of carbapenemase- producing bacteria in a sample and kits and solid supports for use in such methods.

### BACKGROUND ART

Carbapenemases are a group of β-lactamases that are active against various antibiotics including carbapenems. Carbapenemases belong to three classes of beta-lactamases, namely Ambler class A, Ambler class B and Ambler class D. These three classes of carbapenemases confer resistance in clinically important bacteria to carbapenems or decreased susceptibility to carbapenems. Bacteria that produce carbapenemase are therefore of interest clinically and methods for their early detection are required in order to prevent their spread and to reduce multidrug and pandrug resistance.

Two tests are widely used clinically to detect carbapenemase producing bacteria; the "Etest®" and other MIC strip products and the "Modified Hodge-Test". In the "Etest®" indicator carbapenem and carbapenem plus enzyme inhibitor antimicrobial agents against a test microorganism are provided in opposing predefined gradients on agar and used to determine the Minimum Inhibitory Concentration (MIC) of the antimicrobial agent and the inhibitory concentration ratio. In the "Modified Hodge-Test" carbapenemase producing bacteria are detected when reporter bacteria grow towards a carbapenem containing disk to produce a characteristic "clover-leaf" growth pattern around the disk. This phenomenon can be mediated by the carbapenem inactivating enzymes produced by the test isolate. Molecular detection techniques for carbapenemase genes have also been used. However, all three tests have their drawbacks. The "Etest®" and the "Modified Hodge-Test" are neither sensitive nor specific enough whilst molecular detection techniques are complicated and expensive. Moreover, all three detection methods are highly time consuming with up to 24 hours being a typical timescale for determining whether a sample contains carbapenemase producing bacteria. This is unsatisfactory when controlling nosocomial acquired infections and the transfer of drug resistance.

Recently acido-colorimetric techniques have been developed for the detection of carbapenemase producing bacteria which are reported as significantly lowering the assay time compared to "Etest®", "Modified Hodge-test" and molecular detection techniques. WO2012/175637 describes such a method in which specifically, assay times of less than 2 hours are reported. The method described in WO2012/175637 has a cell lysis step before testing can commence. The present invention avoids the need for a lysis step by testing directly from the culture. Some of the methods described in the prior art also require the use of an antibiotic (imipenem) with limited stability in aqueous systems (Viaene et al, Antimicrobial Agents and Chemotherapy, August 2002, pp2327-2332). The present invention overcomes this problem.

US 2014/0134656 discloses a method for detecting the presence of carbapenemase-producing bacteria in a sample, said method comprising the steps of: a) performing cell lysis on a test sample in order to obtain an enzymatic suspension; b) reacting a fraction of the enzymatic suspension obtained in step a) with a reagent kit, said reagent kit comprising a carbapenemase substrate selected from the group consisting of carbapenems and cephamycins, and a pH color indicator which will change color when the pH of the reaction mixture is comprised between 6.4 and 8.4, wherein a color change after step b) indicates the presence of carbapenemase-producing bacteria in the test sample.

WO 2015/013177 discloses methods, reagents, kits, and systems for detecting drug-resistant bacteria, particularly carbapenemase and beta-lactamase producing bacteria. The methods involve contacting unlysated bacterial samples with a test composition including an antibiotic drug composition and a dye and monitoring a color change produced as a result of degradation of the antibiotic drug.

P. Nordmann et al., Emerg Infect Dis. 2012 Sep; 18(9): 1503-1507, discloses a Carba NP test to rapidly identify carbapenemase producers in Enterobacteriaceae. The test uses isolated bacterial colonies and is based on in vitro hydrolysis of a carbapenem, imipenem.

S. AbdelGhani et al., J Clin Microbiol. 2015 Nov; 53(11): 3539-3542, provides a study to compare the Carba NP test to two alternative tests for accuracy and convenience. These were a modified Carba NP test that utilized intravenous (i.v.) imipenem-cilastatin, which is less expensive than reference standard imipenem powder, and an updated version of the Rosco Neo-Rapid Carb kit, which does not require the preparation of imipenem solution and has a shelf life of 2 years.

L. Dortet et al., J Antimicrob Chemother. 2015 Nov;70(11):3014-22, provides a study of the evaluation of the performance of two commercially available biochemical tests for the rapid detection of carbapenemase-producing Enterobacteriaceae compared with a home-made technique.

A. Williams et al., Brit. Soc. Antimicrob Chemother. Poster, 2014 Dec. discloses different commercially available systems for the rapid direct detection of carbapenamase producing *Enterobacteriaceae* and *Pseudomonas aeruginosa* in positive blood cultures.

J.M.T. Hamilton, Pharmacotherapy. 2003 Nov; 23(11):1497-507, discloses chemical and microbiologic aspects of penems, providing a review of beta-lactams including structural hybrids of penicillins (penams) and cephalosporins (cephems). Particular emphasis is placed on faropenem.

R. Wise, Antimicrobic Newsletter, 1990 Oct, 7(10): 73-78 provides a brief review of imipenem, meropenem, LJC 10,627 and FCE 22101, including their structural. antimicrobial, pharmacokinetic and clinical properties.

Nevertheless, there is a need to provide an assay which provides interpretable results in even shorter timescales but which is specific and sensitive and may be easily utilized clinically.

### SUMMARY OF THE INVENTION

The inventors have found that by conducting an acido-colorimetric based assay using a penem substrate it is possible to detect the presence of carbapenemase producing bacteria in very short timescales. In one aspect, the inventors have found that a penem substrate based assay is capable of detecting the presence of carbapenemase producing bacteria in samples containing whole cells thereby avoiding the need to conduct a lysis step which even further improves the assay timescale.

Thus, in a first aspect the present invention provides a method for detecting the presence of carbapenemase-producing bacteria in a sample, comprising:
providing a sample suspected of having carbapenemase producing bacteria; and reacting the sample with a reagent kit comprising:
   a) a carbapenemase substrate which is a penem;
   b) a pH color indicator which will change color when the pH of the reaction mixture is from 5.2 to 8.0; and
   c) a carbapenemase activator
wherein a color change in the reaction medium indicates the presence of carbapenemase-producing bacteria in the test sample.

In a second aspect the present invention provides a reagent kit comprising a carbapenemase substrate which is a penem, a pH color indicator and a carbapenemase activator, wherein the penem, pH colour indicator and carbapenemase activator are as defined in the first aspect of the present invention. Optionally, the reagent kit further comprises a carbapenemase inhibitor.

The invention also relates to a microtitre plate comprising a well or a series of wells comprising a penem substrate and its use in detecting the presence of carbapenemase producers in a test sample.

Thus, in a third aspect, the present invention provides a solid support for use in a method according to the first aspect of the invention, the solid support having one or more reaction areas comprising a carbapenemase substrate which is a penem; a pH indicator which will change colour when the pH of the reaction is from 5.2 to 8.0 and a carbapenemase activator wherein a color change in the reaction medium indicates the presence of carbapenemase-producing bacteria in the test sample. Optionally, the one or more reaction areas may further comprise a carbapenemase inhibitor.

### DETAILED DESCRIPTION OF THE INVENTION

Methods which allow rapid detection of carbapenemase-producing bacteria are needed in order to take necessary action to prevent the spread of antibiotic resistance and to preserve the efficacy of antibiotics such as penems. The method of the present invention addresses this need by providing a user with interpretable results to determine whether a sample has carbapenemase producing bacteria in a fast and reliable manner.

The method of the invention is based on the concept that by hydrolysing the beta- lactam ring of a carbapenemase substrate, such as penem, the carbapenemase generates a carboxyl group which in turn acidifies a medium, typically an unbuffered medium. The acidity resulting from this hydrolysis is then identified by a color change of a pH color indicator. A change in color indicates the presence of a carbapenemase.

In one aspect, the inventors have found that the method of the invention may be conducted on samples having whole cells and that lysis of the cells in the sample is not required in order for any carbapenemase that may be produced by bacteria in the sample to hydrolyse the penem substrate to obtain a detectable pH change in the reaction medium. This finding has advantages over the prior art methods since it reduces the time required to conduct lysis of the sample.

The method of the present invention covers both testing samples which have whole bacterial cells present and have not been lysed and/or samples from a subject in which any bacteria in the sample have been lysed. In one aspect, a bacterial colony may be isolated from a sample obtained from a subject suspected of being infected with carbapenem producing bacteria. The colony may then be inoculated into a culture broth or onto culture medium and cultured. The bacterial culture may then be tested in the method of invention as whole cells or lysed in a lysis buffer before being tested in the method of the invention.

A small fraction of whole cell sample or the lysed sample is mixed with the reagent kit of the invention comprising a carbapenemase substrate and a pH color indicator. Preferably, a "fraction" according to the invention is from 1µl to 100 µl of whole cell sample or lysed sample.

Typically, the method of the present invention is conducted on samples obtained from a subject which is suspected of being infected with carbapenemase producing bacteria. The sample may be any biological sample obtained from a subject such as fluids, tissues or cell samples. Preferably the sample is a whole blood, serum, plasma or urine sample. The sample may be obtained by known methods from the subject which may be a mammal. Preferably the subject from which the sample is obtained is a human.

The method of the present invention may be used to detect any carbapenemase-producing bacteria. The carbapenemase-producing bacteria may be gram positive or gram negative bacteria. Preferably, the carbapenemase-producing bacteria are of clinical importance such as bacteria that are responsible for nosocomial or community-acquired infections.

More preferably, the carbapenemase-producing bacteria are selected from the genera consisting of *Acinetobacter, Aeromonas, Bacillus, Bacteroides, Pseudomonas, Ralstonia, Stenotrophomonas, Citrobacter, Enterobacter, Escherichia, Klebsiella, Morganella, Proteus, Providencia, Raoultella, Salmonella, Serratia* and *Shigella.*

Preferably, the carbapenemase-producing bacteria are selected from the group consisting of *Acinetobacter baumanii, Bacillus cereus, Bacteroides fragilis, Citrobacter amalonaticus, Citrobacter freundii, Enterobacter aerogenes, Enterobacter cloacae, Escherichia coli, Klebsiella oxytoca, Klebsiella pneumoniae, Morganella morganii, Proteus mirabilis, Proteus vulgaris, Providencia rettgeri, Providencia stuartii, Pseudomonas aeruginosa, Salmonella enterica, Serratia marcescens, Shigella flexneri, Stenotrophomonas maltophila and Ralstonia pickettii.*

The carbapenemase substrate used in the present invention is a penem. Preferably, the penem may be selected from the group consisting of faropenem, sulopenem and ritipenem.

Typically, the concentration of carbapenemase substrate used in the reagent kit is from 0.1 mg/ml to 10 mg/ml, more preferably from 1 mg/ml to 5 mg/ml and even more preferably from 2 mg/ml to 3 mg/ml.

The pH color indicator used in the method of the present invention changes colour when the pH of the reaction mixture is from 5.2 to 8.0. The skilled person is well aware from his general knowledge of suitable pH indicators that change colour in the pH range of from 5.2 to 8.0. Preferably, the pH indicator in selected from phenol red, bromothymol blue and bromocresol purple. Phenol red has a transition range of from pH 6.4 to pH 8.0. bromothymol blue has a transition range of from pH 6.0 to pH 7.6, bromocresol purple has a transition range of from pH 5.2 to pH 6.8.

Typically, the concentration of pH indicator used in the reagent kit is from 0.01% to 1%, more preferablyfrom 0.03% to 0.08% and more preferably from 0.04% to 0.05% for bromothymol blue or bromocresol purple. The concentration of pH indicator used in the reagent kit is from 0.01% to 1%, more preferably from 0.4% to 0.8% and even more preferably from 0.5% to 0.6% for phenol red.

The reagent kit may comprise a carbapenemase activator to act as a cofactor to improve enzyme performance. The activator is not always necessary since some classes of carbapenemase exhibit hydrolytic activity without the need for a cofactor. If an activator is used then the cofactor is preferably a divalent cation. More preferably the divalent cation is a salt of manganese, cobalt, nickel, cadmium, mercury, zinc and mixtures thereof. A zinc salt, such as zinc sulphate, is the preferred activator. In some embodiments the carbapenemase activator is used at a concentration of from 0.01 mM to 1mM, preferably 0.1mM to 0.5mM and more preferably from 0.1mM to 0.2mM.

The reaction of the sample, either whole cells or the fraction of lysed sample, with a reagent kit is conducted over a period of time sufficient to observe a color change in the pH indicator that indicates the presence of the carbapenem producing bacteria. Typically the colour change is observed less than 2 hours, preferably less than 1 hour and more preferably less than 30 minutes from mixing the sample with whole cells or the fraction with the reagent. In the case where the method is conducted on samples comprising whole cells which are not lysed then the assay times are much quicker than prior art methods in which lysis of the sample is needed.

Typically, the reaction of the sample either whole cells or the fraction of lysed sample, with a reagent kit is carried out at a temperature comprised from 15°C to 40°C, preferably from 20°C to 37°C, more preferably from 35°C to 37°C.

The method of the invention provides a very rapid way of identifying an infection with a carbapenemase producing bacteria. Different classes of carbapenemase may be inhibited by different kinds of inhibitors which are known to the skilled person (Queenan et al, Clinical Microbiology Reviews, July 2007, pp440-458) Once the presence of an infection by a carbapenemase producing bacteria has been identified then the type of bacteria may be further characterised using inhibitors that are specific for a particular type of carbapenemase producing bacteria or by testing with PCR and sequenced for identification of carbapenemase genes.

In a second aspect the present invention provides a reagent kit comprising a carbapenemase substrate which is penem and a pH colour indicator and a carbapenemase activator. According to an embodiment of the invention, the reagent kit may further comprise a carbapenemase inhibitor. The carbapenemase substrate, pH color indicator, carbapenemase activator and carbapenemase inhibitor are as defined previously in the present patent application. Embodiments of the method of the invention in which a carbapenemase inhibitor are used allow the specific types of carbapenemase producing bacteria present in the sample to be identified.

The reagent kit of the invention is a product having a plurality of components. These components comprise a carbapenemase substrate which is penem, a pH colour indicator and optionally, a carbapenemase activator and a carbapenemase inhibitor. The components may be used separately, simultaneously or sequentially in the method of the invention.

The reagent kit is used to detect the presence of carbapenemase-producing bacteria in a sample according to the method of the present invention.

In a third aspect, the present invention provides a solid support upon which a method of the invention may be conducted, having one or more reaction areas comprising a carbapenemase substrate which is a penem. Preferably, the reagent kit as described in more detail above comprising a carbapenemase substrate which is a penem, a pH indicator and a carbapenemase activator may be incorporated into the reaction area. In some embodiments the solid support may be an absorbent material on to which the reagent kit may be absorbed to form the one or more reaction areas. The method of the invention may be conducted on the solid support by simply bringing a culture from a test sample obtained from a person suspected of having an infection of carbapenemase producing bacteria into contact with a test area. A colour change at the reaction area is indicative of the presence of carbapenemase producing bacteria in the sample.

Preferably the solid support is microtitre plate in which the one or more reaction areas is one or more wells comprising a carbapenemase substrate which is a penem. The microtitre plate may also contain a control well or a series of control wells which can be assayed and compared to the test samples. Typically, the microtitre plate is a 96-well microtitre plate. One or more of the components of the reagent kit may be directly coated or bound to the surface walls of each well of the microtitre plate or to the wall of a microtube.

According to an embodiment, the microtitre plate as well as each of the elements used to perform the method of the invention may be enclosed within an individual container and all of the various containers may be placed within a single package along with instructions for observing whether carbapenemase-producing bacteria can be found in the test sample.

The invention will further be illustrated in view of the following and examples.

### Examples

The following solutions were made and 100µl each solution was dispensed into 96 well microtitre plates.
Solution 1: 3mg/ml faropenem, 0.1mmol/L zinc sulphate and 0.04% bromothymol blue solution
Solution 2: 3mg/ml faropenem, 0.1mmol/L zinc sulphate and 0.04% bromocresol purple solution
Solution 3: 3mg/ml faropenem, 0.1mmol/L zinc sulphate and 0.5% phenol red solution

The panel of organisms used in this test is shown in table 1.

**Table 1: Panel of organisms**

| **Organism** | **Mast Code** | **Resistance** |
|---|---|---|
| *Klebsiella pneumoniae* | KI130 | OXA |
| *Escherichia coli* | Ec202 | KPC |
| *Escherichia coli* | Ec243 | MBL |
| *Klebsiella pneumoniae* | KI104 | KPC |
| *Klebsiella pneumoniae* | KI97 | MBL |

The organisms tested are available from Mast Group Limited.

Organisms were tested as whole cells by putting 5µl of cells directly into the faropenem indicator solutions.

Organisms were also tested as lysed cells. Cells were extracted using tris buffer and then 30µl of the supernatant was added to the faropenem indicator solutions. The positive control is the faropenem containing solution. The negative control is the pH indicator without antibiotic.

These results are shown in Table 2. As can be seen, after 2 hours of incubation of test organisms on a penem substrate an identifiable colour change was observed which indicated the presence of carbapenemase producing bacteria. The colour change was observed in both lysed cells and whole cell samples. This indicates that the method of the present invention may be used on whole cells thereby avoiding the need to conduct a lysis step.

After 30 minutes incubation, the colour reaction for bromocresol purple was a much stronger yellow.

### Results of Colorimetric Test

| **Organism** | **Mast Code** | **Resistance** | **Colorimetric Test Results at 2 hours incubation** |
|---|---|---|---|
| *Klebsiella pneumoniae* | KI130 | OXA | + |
| *Escherichia coli* | Ec202 | KPC | + |
| *Escherichia coli* | Ec243 | MBL | + |
| *Klebsiella pneumoniae* | KI104 | KPC | + |
| *Klebsiella pneumoniae* | KI97 | MBL | + |

## Claims

1. A method for detecting the presence of carbapenemase-producing bacteria in a sample, comprising:
providing a sample suspected of having carbapenemase-producing bacteria; and
reacting the sample with a reagent kit comprising:
a) a carbapenemase substrate which is a penem;
b) a pH color indicator which will change color when the pH of the reaction mixture is from 5.2 to 8.0,
c) a carbapenemase activator,
wherein a color change in the reaction medium indicates the presence of carbapenemase-producing bacteria in the test sample.

2. The method according to claim 1, further comprising a step of lysing and reacting the lysate with the reagent kit.

3. The method according to claim 1, wherein the sample comprises whole cells.

4. The method according to any preceding claim, wherein the test sample is a biological sample selected from the group consisting of a blood sample and a urine sample.

5. The method according to any preceding claim, wherein the carbapenemase-producing bacteria are selected from the genera consisting of *Acinetobacter*, *Aeromonas*, *Bacillus, Bacteroides*, *Pandoreae*, *Pseudomonas, Ralstonia, Shewanella, Strenotrophomonas, Citrobacter, Enterobacter, Escherichia, Klebsiella, Morganella, Proteus, Providencia, Raoultella, Salmonella, Serratia* and *Shigella* and preferably selected from *Acinetobacter baumanii, Aeromonas junii, Bacillus cereus, Bacteroides fragilis, Citrobacter amalonaticus, Citrobacter freundii, Citrobacter youngae, Enterobacter aerogenes, Enterobacter asburiae, Enterobacter cloacae, Escherichia coli, Klebsiella oxytoca, Klebsiella pneumoniae, Morganella morganii, Pandoraea pnomenusa, Proteus mirabilis, Proteus rettgeri, Proteus vulgaris, Providencia stuartii, Pseudomonas aeruginosa, Salmonella enterica, Serratia marcescens, Shigella flexneri, Stenotrophomonas maltophila, Ralstonia picketti* and *Shewanella algae.*

6. The method according to any preceding claim, wherein the reaction of the sample with the reagent kit is carried out at a temperature of from 15°C to 40°C, preferably from 20°C to 37°C, more preferably from 35°C to 37°C.

7. The method according to any preceding claim, wherein the reaction of the sample with the reagent kit is carried out over a period of time sufficient to observe a color change, preferably less than 2 hours, more preferably less than 1 hour and most preferably less than 30 minutes from mixing the sample with the reagent kit.

8. The method of any preceding claim, wherein the pH indicator is selected from phenol red, bromothymol blue and bromocresol purple.

9. The method of any preceding claim, wherein the penem is selected from faropenem, sulopenem and ritipenem.

10. The method of any preceding claim, wherein the carbapenemase activator is selected from the group consisting of divalent cations or salts thereof, and mixtures thereof and wherein optionally the method, further comprises a carbapenemase inhibitor.

11. A reagent kit comprising a carbapenemase substrate which is a penem, a pH colour indicator and a carbapenemase activator, wherein the penem, pH colour indicator and carbapenemase activator are as defined in any of claims 1 to 10; wherein optionally the reagent kit, further comprises a carbapenemase inhibitor.

12. A solid support for use in a method according to any of claims 1 to 10, the solid support having one or more reaction areas comprising a carbapenemase substrate which is a penem; wherein the one or more reaction areas further comprises a pH indicator which will change color when the pH of the reaction mixture is from 5.2 to 8.0 and a carbapenemase activator wherein a color change in the reaction medium indicates the presence of carbapenemase-producing bacteria in the test sample, wherein the one or more reaction areas optionally further comprises a carbapenemase inhibitor.

13. The solid support of claim 12, which is a microtitre plate comprising a well or a series of wells as the one or more reaction areas or is a microtube.

## Patentansprüche

1. Verfahren für den Nachweis der Gegenwart von Carbapenemase-produzierenden Bakterien in einer Probe, das Folgendes umfasst:
Bereitstellen einer Probe, von der vermutet wird, dass sie Carbapenemaseproduzierende Bakterien aufweist; und
Reagieren der Probe mit einem Reagenz-Kit, der Folgendes umfasst:
a) ein Carbapenemase-Substrat, das ein Penem ist;
b) einen pH-Farbindikator, der die Farbe ändern wird, wenn der pH der Reaktionsmischung von 5,2 bis 8,0 beträgt,
c) ein Carbapenemase-Aktivierungsmittel,
wobei eine Farbänderung in dem Reaktionsmedium die Gegenwart von Carbapenemase-produzierenden Bakterien in der Untersuchungsprobe anzeigt.

2. Verfahren nach Anspruch 1, das weiter einen Schritt des Lysierens und des Reagierens des Lysats mit dem Reagenz-Kit umfasst.

3. Verfahren nach Anspruch 1, wobei die Probe ganze Zellen umfasst.

4. Verfahren nach einem vorstehenden Anspruch, wobei die Untersuchungsprobe eine biologische Probe ist, die aus der Gruppe ausgewählt ist, die aus einer Blutprobe und einer Urinprobe besteht.

5. Verfahren nach einem vorstehenden Anspruch, wobei die Carbapenemase-produzierenden Bakterien aus den Gattungen ausgewählt sind, die aus Folgenden bestehen: *Acinetobacter*, *Aeromonas, Bacillus, Bacteroides*, *Pandoreae, Pseudomonas, Ralstonia, Shewanella, Stenotrophomonas, Citrobacter, Enterobacter, Escherichia, Klebsiella, Morganella, Proteus, Providencia, Raoultella*, *Salmonella*, *Serratia* und *Shigella,* und bevorzugt aus Folgenden ausgewählt sind: *Acinetobacter baumanii, Aeromonas junii, Bacillus cereus, Bacteroides fragilis, Citrobacter amalonaticus, Citrobacter freundii, Citrobacter youngae, Enterobacter aerogenes, Enterobacter asburiae, Enterobacter cloacae, Escherichia coli, Klebsiella oxytoca, Klebsiella pneumoniae, Morganella morganii, Pandoraea pnomenusa, Proteus mirabilis, Providencia rettgeri, Proteus vulgaris, Providencia stuartii, Pseudomonas aeruginosa, Salmonella enterica, Serratia marcescens, Shigella flexneri, Stenotrophomonas maltophila, Ralstonia picketti* und *Shewanella algae.*

6. Verfahren nach einem vorstehenden Anspruch, wobei die Reaktion der Probe mit dem Reagenz-Kit bei einer Temperatur von 15 °C bis 40 °C, bevorzugt von 20 °C bis 37 °C, bevorzugter von 35 °C bis 37 °C durchgeführt wird.

7. Verfahren nach einem vorstehenden Anspruch, wobei die Reaktion der Probe mit dem Reagenz-Kit über eine Zeitdauer durchgeführt wird, die zur Beobachtung einer Farbänderung ausreicht, bevorzugt weniger als 2 Stunden, bevorzugter weniger als 1 Stunde und am bevorzugtesten weniger als 30 Minuten nach dem Mischen der Probe mit dem Reagenz-Kit.

8. Verfahren nach einem vorstehenden Anspruch, wobei der pH-Indikator aus Phenolrot, Bromthymolblau und Bromkresolpurpur ausgewählt ist.

9. Verfahren nach einem vorstehenden Anspruch, wobei das Penem aus Faropenem, Sulopenem und Ritipenem ausgewählt ist.

10. Verfahren nach einem vorstehenden Anspruch, wobei das Carbapenemase-Aktivierungsmittel aus der Gruppe ausgewählt ist, die aus zweiwertigen Kationen oder Salzen davon und Mischungen davon besteht, und wobei optional das Verfahren weiter einen Carbapenemase-Inhibitor umfasst.

11. Reagenz-Kit, der Folgendes umfasst: ein Carbapenemase-Substrat, das ein Penem ist, einen pH-Farbindikator und ein Carbapenemase-Aktivierungsmittel, wobei das Penem, der pH-Farbindikator und das Carbapenemase-Aktivierungsmittel wie in einem der Ansprüche 1 bis 10 definiert sind; wobei optional der Reagenz-Kit weiter einen Carbapenemase-Inhibitor umfasst.

12. Fester Träger für die Verwendung in einem Verfahren nach einem der Ansprüche 1 bis 10, wobei der feste Träger eine oder mehrere Reaktionsflächen aufweist, die ein Carbapenemase-Substrat umfassen, das ein Penem ist; wobei die eine oder mehreren Reaktionsflächen weiter Folgendes umfassen: einen pH-Indikator, der die Farbe ändern wird, wenn der pH der Reaktionsmischung von 5,2 bis 8,0 beträgt, und ein Carbapenemase-Aktivierungsmittel, wobei eine Farbänderung in dem Reaktionsmedium die Gegenwart von Carbapenemase-produzierenden Bakterien in der Untersuchungsprobe anzeigt, wobei die eine oder mehreren Reaktionsflächen optional weiter einen Carbapenemase-Inhibitor umfassen.

13. Fester Träger nach Anspruch 12, der eine Mikrotiterplatte ist, die ein Well oder eine Reihe von Wells als die eine oder mehreren Reaktionsflächen umfasst, oder ein Mikroröhrchen ist.

## Revendications

1. Méthode de détection de la présence de bactéries productrices de carbapénémase dans un échantillon, comprenant :
la mise à disposition d'un échantillon suspecté comme contenant des bactéries productrices de carbapénémase ; et
la réaction de l'échantillon avec un kit de réactifs comprenant :
a) un substrat de carbapénémase qui est un pénème ;
b) un agent colorimétrique indicateur de pH dont la couleur changera lorsque le pH du mélange réactionnel ira de 5,2 à 8,0 ;
c) un activateur de carbapénémase ;
où un changement de couleur dans le mélange réactionnel indique la présence de bactéries productrices de carbapénémase dans l'échantillon à tester.

2. Méthode selon la revendication 1, comprenant en outre une étape consistant à lyser et réagir le lysat avec le kit de réactifs.

3. Méthode selon la revendication 1, dans laquelle l'échantillon comprend des cellules entières.

4. Méthode selon l'une quelconque des revendications précédentes, dans laquelle l'échantillon à tester est un échantillon biologique choisi dans le groupe constitué par un échantillon de sang et un échantillon d'urine.

5. Méthode selon l'une quelconque des revendications précédentes, dans laquelle les bactéries productrices de carbapénémase sont choisies parmi les genres constitués par *Acinetobacter, Aeromonas, Bacillus, Bacteroides, Pandoraea, Pseudomonas, Ralstonia, Shewanella, Stenotrophomonas, Citrobacter, Enterobacter, Escherichia, Klebsiella, Morganella, Proteus, Providencia, Raoultella, Salmonella, Serratia* et *Shigella,* et préférablement choisies parmi *Acinetobacter baumannii, Aeromonasjunii, Bacillus cereus, Bacteroides fragilis, Citrobacter amalonaticus, Citrobacter freundii, Citrobacter youngae, Enterobacter aerogenes, Enterobacter asburiae, Enterobacter cloacae, Escherichia coli, Klebsiella oxytoca, Klebsiella pneumoniae, Morganella morganii, Pandoraea pnomenusa, Proteus mirabilis, Proteus rettgeri, Proteus vulgaris, Providencia stuartii, Pseudomonas aeruginosa, Salmonella enterica, Serratia marcescens, Shigella flexneri, Stenotrophomonas maltophilia, Ralstonia pickettii* et *Shewanella algae.*

6. Méthode selon l'une quelconque des revendications précédentes, dans laquelle la réaction de l'échantillon avec le kit de réactifs est effectuée à une température allant de 15°C à 40°C, préférablement de 20°C à 37°C, plus préférablement de 35°C à 37°C.

7. Méthode selon l'une quelconque des revendications précédentes, dans laquelle la réaction de l'échantillon avec le kit de réactifs est effectuée sur une période de temps suffisante pour observer un changement de couleur, préférablement moins de 2 heures, plus préférablement moins de 1 heure et tout préférablement moins de 30 minutes après le mélange de l'échantillon avec le kit de réactifs.

8. Méthode selon l'une quelconque des revendications précédentes, dans laquelle l'indicateur de pH est choisi parmi le rouge de phénol, le bleu de bromothymol et le pourpre de bromocrésol.

9. Méthode selon l'une quelconque des revendications précédentes, dans laquelle le pénème est choisi parmi le faropénème, le sulopénème, et le ritipénème.

10. Méthode selon l'une quelconque des revendications précédentes, dans laquelle l'activateur de carbapénémase est choisi dans le groupe constitué par les cations divalents ou des sels de ceux-ci, et des mélanges de ceux-ci et où éventuellement la méthode comprend en outre un inhibiteur de carbapénémase.

11. Kit de réactifs, comprenant un substrat de carbapénémase qui est un pénème, un agent colorimétrique indicateur de pH et un activateur de carbapénémase, dans lequel le pénème, l'agent colorimétrique indicateur de pH et l'activateur de carbapénémase sont tels que définis selon l'une quelconque des revendications 1 à 10, où éventuellement le kit de réactifs comprend en outre un inhibiteur de carbapénémase.

12. Support solide destiné à une utilisation dans une méthode selon l'une quelconque des revendications 1 à 10, le support solide présentant une ou plusieurs zones de réaction comprenant un substrat de carbapénémase qui est un pénème ; dans lequel les une ou plusieurs zones de réaction comprennent en outre un indicateur de pH dont la couleur changera lorsque le pH du mélange réactionnel ira de 5,2 à 8,0 et un activateur de carbapénémase, où un changement de couleur dans le mélange réactionnel indique la présence de bactéries productrices de carbapénémase dans l'échantillon à tester, où les une ou plusieurs zones de réaction comprennent éventuellement en outre un inhibiteur de carbapénémase.

13. Support solide selon la revendication 12, qui est une plaque de microtitration comprenant un puits ou une série de puits représentant les une ou plusieurs zones de réaction ou est un microtube.
